# EUROPÄISCHE PATENTSCHRIFT

(11) **EP 0 338 494 B1**
(45) Veröffentlichungstag und Bekanntmachung des Hinweises auf die Patenterteilung: **10.01.1996**
(21) Anmeldenummer: 89106896.7
(22) Anmeldetag: 18.04.1989
(51) Int. Cl.: C07D 333/38

(54) **3-Anilino-2-hydroxycarbonyl-4-thiophenessigsäuren**
3-Anilino-2-hydroxycarbonyl-4-thiophen-acetic acids
Acides 3-anilino-2-hydroxycarbonyl-4-thiophène-acétiques

(30) Priorität: 22.04.1988 DE 3813644
(43) Veröffentlichungstag der Anmeldung: 25.10.1989
(73) Patentinhaber: Byk Gulden Lomberg Chemische Fabrik GmbH, D-78403 Konstanz (DE)
(72) Erfinder: Figala, Volker, Dr., D-7753 Allensbach 4 (DE)

(56) Entgegenhaltungen:
- EP-A- 0 005 559
- EP-A- 0 210 320
- GB-A- 2 132 607
- CHEMICAL ABSTRACTS, Band 78, Nr. 25, 25. Juni 1973, Seite 400, Zusammenfassung Nr. 159416x, Columbus, Ohio, US; & SU-A-364 612

## Beschreibung

### Anwendungsgebiet der Erfindung

Die Erfindung betrifft neue 3-Anilino-2-hydroxycarbonyl-4-thiophenessigsäuren, Verfahren zu ihrer Herstellung und ihre Verwendung zur Synthese von pharmazeutischen Wirkstoffen, die in der pharmazeutischen Industrie zur Herstellung von Arzneimitteln eingesetzt werden.

### Bekannter technischer Hintergrund

Im europäischen Patent Nr. 5559 wird die Herstellung bestimmter Phenylaminothiophenessigsäurederivate beschrieben, die als Wirkstoffe in Arzneimitteln Verwendung finden sollen.

### Beschreibung der Erfindung

Gegenstand der Erfindung sind sowohl ein neues Verfahren zur Herstellung bestimmter Phenylaminothiophenessigsäurederivate als auch neue Zwischenprodukte, die in diesem Verfahren eingesetzt werden.

Ein erster Aspekt der Erfindung sind somit neue 3-Anilino-2-hydroxycarbonyl-4-thiophenessigsäuren der Formel I 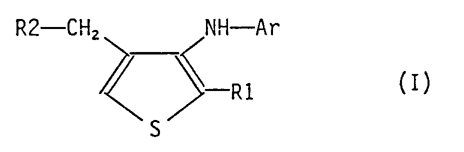
worin
- R1: -CO-O-R6 bedeutet,
- R2: -CO-O-R7 oder ein sonstiges funktionelles Carbonsäurederivat G bedeutet,
- Ar: den Rest 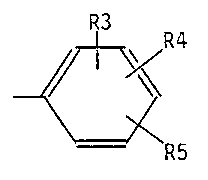 bedeutet,
- R3: Wasserstoff, Halogen, 1-4C-Alkyl, 1-4C-Alkoxy oder Trifluormethyl bedeutet,
- R4: Wasserstoff, Halogen, 1-4C-Alkyl oder 1-4C-Alkoxy bedeutet,
- R5: Wasserstoff, Halogen, 1-4C-Alkyl oder 1-4C-Alkoxy bedeutet,
- R6: Wasserstoff oder 1-4C-Alkyl bedeutet und
- R7: Wasserstoff, 1-4C-Alkyl oder Hydroxy-1-4C-alkoxy-1-4C-alkyl bedeutet,
und die Salze dieser Verbindungen.

Ein funktionelles Carbonsäurederivat G ist eine Gruppe, aus der durch Solvolyse, Thermolyse oder Hydrogenolyse die Carboxylgruppe oder die Gruppe -CO-O-R7 hergestellt werden kann. Welche Bedeutungen G dabei beispielsweise haben kann, und unter welchen Bedingungen die Gruppe G solvolysiert, thermolysiert oder hydrogenolysiert werden kann, ist im europäischen Patent 5559 beschrieben.

Halogen im Sinne der vorliegenden Erfindung ist Jod, Brom, Fluor oder insbesondere Chlor.

1-4C-Alkyl steht für die geradkettigen oder verzweigten Reste t-Butyl, i-Butyl, sec.-Butyl, Butyl, Isopropyl, Propyl und insbesondere Ethyl und Methyl.

1-4C-Alkoxy enthält neben dem Sauerstoffatom einen der vorstehend genannten 1-4C-Alkylreste. Bevorzugt sind der Ethoxy- und der Methoxyrest.

Als bevorzugte Hydroxy-1-4C-alkoxy-1-4C-alkylgruppe ist die 2-Hydroxy-ethoxyethylgruppe zu nennen.

Als beispielhafte Reste Ar seien die Reste 2-Trifluormethylphenyl, 3-Trifluormethylphenyl, 4-Trifluormethylphenyl, 2-Chlor-5-trifluormethylphenyl, 4-Chlor-3-trifluormethylphenyl, 2,4-Dimethylphenyl, 2,5-Dimethylphenyl, 2-Methoxyphenyl, 3-Methoxyphenyl, 4-Methoxyphenyl, 2-Bromphenyl, 3-Bromphenyl, 4-Bromphenyl, 4-Brom-2-chlorphenyl, 4-Brom-2,6-dimethylphenyl, 2-Brom-4-methylphenyl, 4-Brom-2-methylphenyl, 4-Brom-3-methylphenyl, 6-Brom-3-chlor-2-methylphenyl, 2-Chlorphenyl, 3-Chlorphenyl, 4-Chlorphenyl, 2-Chlor-5-methoxyphenyl, 5-Chlor-2,4-dimethoxyphenyl, 2-Chlor-4-methylphenyl, 2-Chlor-6-methylphenyl, 3-Chlor-4-methylphenyl, 3-Chlor-2-methylphenyl, 4-Chlor-2-methylphenyl, 5-Chlor-2-methylphenyl, 2,4-Dibromphenyl, 2,5-Dibromphenyl, 2,6-Dibrom-4-methylphenyl, 2,3-Dichlorphenyl, 2,4-Dichlorphenyl, 2,5-Dichlorphenyl, 2,6-Dichlorphenyl, 3,4-Dichlorphenyl, 3,5-Dichlorphenyl, 3,4-Diethoxyphenyl, 2,6-Diethylphenyl, 2,4-Difluorphenyl, 2,6-Difluorphenyl, 2,6-Diisopropylphenyl, 2,4-Dimethoxyphenyl, 2,5-Dimethoxyphenyl, 3,5-Dimethoxyphenyl, 2,3-Dimethylphenyl, 2,6-Dimethylphenyl, 3,4-Dimethylphenyl, 3,5-Dimethylphenyl, 2-Ethylphenyl, 3-Ethylphenyl, 4-Ethylphenyl , 2-Fluorphenyl, 3-Fluorphenyl, 4-Fluorphenyl, 5-Fluor-2-methylphenyl, 2-Jodphenyl, 3-Jodphenyl, 4-Jodphenyl, 2-Methoxy-5-methylphenyl, 4-Methoxy-2-methylphenyl, 3-Methoxy-5-trifluormethylphenyl, 2-Methylphenyl, 3-Methylphenyl, 4-Methylphenyl, 2,4,6-Tribromphenyl, 2,3,4-Trichlorphenyl, 2,4,5-Trichlorphenyl, 2,4,6-Trichlorphenyl, 3,4,5-Trichlorphenyl, 2,4,6-Trifluorphenyl, 3,4,5-Trimethoxyphenyl und 2,4,6-Trimethylphenyl genannt.

Als Salze kommen alle herstellbaren Salze, insbesondere die Salze der Säuren in Frage. Hier seien beispielsweise die Alkalimetall- (z.B. Natrium-, Kalium-), Erdalkalimetall- (z.B. Calcium-, Magnesium-) oder Erdmetall- (z.B. Aluminium-) -salze genannt.

Bevorzugte Verbindungen der Formel I sind solche, in denen
- R1: -CO-O-R6 bedeutet,
- R2: -CO-O-R7 bedeutet,
- Ar: 2,6-Dichlorphenyl, 2,6-Difluorphenyl, 2-Chlor-6-methylphenyl, 6-Brom-3-chlor-2-methylphenyl, 3-Chlor-2-methylphenyl, 2,6-Dichlor-3-methylphenyl, 2-Chlor-3-methylphenyl, 2,6-Dimethylphenyl, 2-Chlorphenyl, 4-Methoxy-2-methylphenyl oder 3-Trifluormethylphenyl bedeutet,
- R6: Wasserstoff, Methyl oder Ethyl bedeutet und
- R7: Wasserstoff, Methyl, Ethyl oder 2-Hydroxy-ethoxy-ethyl bedeutet,
und ihre Salze.

Besonders bevorzugte Verbindungen der Formel I sind solche, in denen
- R1: -CO-O-R6 bedeutet,
- R2: -CO-O-R7 bedeutet,
- Ar: 2,6-Dichlorphenyl bedeutet,
- R6: Wasserstoff, Methyl oder Ethyl bedeutet und
- R7: Wasserstoff, Methyl, Ethyl oder 2-Hydroxy-ethoxy-ethyl bedeutet,
und ihre Salze.

Die Verbindungen der Formel I und ihre Salze sind wertvolle Zwischenprodukte zur Herstellung der im europäischen Patent 5559 beschriebenen Phenylaminothiophenessigsäurederivate der Formel II, 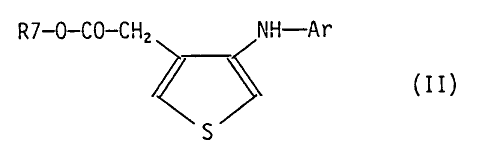
worin Ar und R7 die obengenannten Bedeutungen haben. Die Erfindung betrifft somit in einem weiteren Aspekt die Verwendung der Verbindungen der Formel I in einem Verfahren zur Herstellung von Verbindungen der Formel II und ihren Salzen.

Das Verfahren ist dadurch gekennzeichnet, daß man Verbindungen der Formel I decarboxyliert oder verseift und anschließend decarboxyliert. Vor oder nach der Decarboxylierung kann der Substituent R2 durch Solvolyse, Thermolyse oder Hydrogenolyse auf übliche Weise in einen anderen gewünschten Substituenten R2 überführt werden.

Die Decarboxylierung erfolgt durch Erhitzen, beispielsweise auf Temperaturen zwischen 50°C und 100°C, in saurem, vorzugsweise wäßrigem Medium, beispielsweise in 2N Salzsäure.

Eine Verseifung vor der Decarboxylierung ist erforderlich, wenn R6 1-4C-Alkyl bedeutet. Diese Verseifung wird in an sich bekannter Weise, bevorzugt unter Verwendung einer Lauge, wie beispielsweise Natronlauge oder Kalilauge, bei Temperaturen zwischen 50° und 100°C und anschließender saurer Aufarbeitung vorgenommen.

Bei der Verseifung etwaiger Esterreste R1 werden gegebenenfalls auch vorhandene Esterreste R2 oder - je nach Art des funktionellen Carbonsäurederivates G - gegebenenfalls auch vorhandene Reste G in den Carboxylrest überführt. Eine besonders bevorzugtes Verfahren ist daher die Decarboxylierung von Verbindungen der Formel I, worin
- R1: -CO-O-R6 bedeutet,
- R2: -CO-O-R7 bedeutet,
- Ar: 2,6-Dichlorphenyl bedeutet und
- R6 und R7: Wasserstoff bedeuten.

Ein weiterer Gegenstand der Erfindung ist ein Verfahren zur Herstellung der neuen Verbindungen der Formel I. Das Verfahren ist dadurch gekennzeichnet, daß man Verbindungen der Formel III 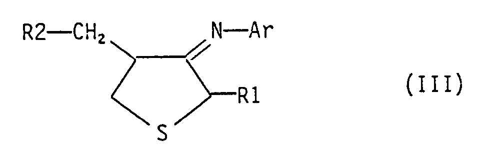
dehydriert. Vor oder nach der Dehydrierung können die Substituenten R1 und/oder R2 durch Solvolyse, Thermolyse oder Hydrogenolyse auf übliche Weise in andere gewünschte Substituenten R1 oder R2 überführt werden.

Die Dehydrierung (Oxidation) der Verbindungen III kann mit geeigneten Reagenzien, z.B. mit Chinonen, wie Chloranil, oder mit Halogenen, wie Chlor oder insbesondere Brom, oder mit N-Bromsuccinimid (NBS) in für das Oxidationsmittel zweckmäßigen, inerten Lösungsmitteln durchgeführt werden. Als Lösungsmittel eignen sich beispielsweise Ether, wie Dioxan, Diethylether, Tetrahydrofuran, Glykolmonoethylether oder Glykoldimethylether; Alkohole, wie Ethanol oder Methanol; aromatische Kohlenwasserstoffe, wie Xylol oder Toluol; oder chlorierte Kohlenwasserstoffe, wie Methylenchlorid, Chloroform, Tetrachlorethan oder Dichlorethan; oder polare aprotische Lösungsmittel, wie Dimethylformamid oder Dimethylsulfoxid.

Die Reaktionstemperaturen können - je nach Reaktivität des Oxidationsmittels - in einem weiten Bereich variieren. Im allgemeinen wird die Umsetzung bei Temperaturen zwischen -70°C und 80°C, vorzugsweise zwischen -20°C und +20°C, insbesondere bei Temperaturen um oder unterhalb 0°C durchgeführt.

Die Isolierung und Reinigung der erhaltenen Verbindungen I erfolgt in an sich bekannter Weise z.B. derart, daß man das Lösungsmittel, gewünschtenfalls nach vorheriger Freisetzung der Verbindung I aus ihrem Salz, z. B. durch Versetzen mit Wasser und Abtrennen der wäßrigen Phase, im Vakuum abdestilliert und den erhaltenen Rückstand aus einem geeigneten Lösungsmittel umkristallisiert oder einer der üblichen Reinigungsmethoden unterwirft.

Die Verbindungen der Formel III werden hergestellt, indem man Ketone der Formel IV, 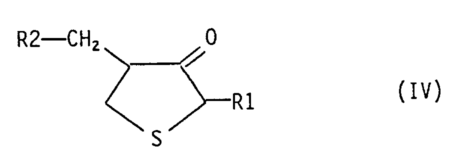
worin R1 und R2 die oben angegebenen Bedeutungen haben, als solche oder in Form ihrer Ketale mit Anilinen der Formel V, 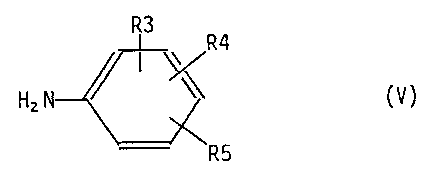
worin R3, R4 und R5 die oben angegebenen Bedeutungen haben, umsetzt und gewünschtenfalls anschließend die freien Verbindungen in die Salze überführt.

Die Umsetzung der Ketone IV bzw. ihrer Ketale mit den Anilinen V wird auf eine Weise vorgenommen, wie sie für die Herstellung von Anilen dem Fachmann vertraut ist. Die Umsetzung erfolgt ohne Lösungsmittel oder in einem inerten, vorzugsweise höhersiedenden Lösungsmittel, beispielsweise in einem Ether, wie Dioxan, Glykolmonoethylether oder Glykoldimethylether, oder in einem aromatischen Kohlenwasserstoff, wie Xylol oder Toluol.

Die Reaktionstemperaturen liegen im allgemeinen zwischen 50°C und 200°C, insbesondere bei der Siedetemperatur des verwendeten Lösungsmittels oder - wenn ohne Lösungsmittel gearbeitet wird - zwischen 120°C und 160°C.

Die Ketone IV werden hergestellt durch Umsetzung von Itaconsäurederivaten VI, 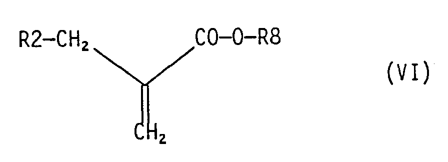
worin R2 -CO-O-R7 oder ein sonstiges geeignetes Carbonsäurederivat G bedeutet, und R7 und R8 vorzugsweise 1-4C-Alkyl bedeuten, mit Thioglykolsäureestern VII,

HS-CH₂-R1 (VII)

worin R1 -CO-O-R6 und R6 1-4C-Alkyl bedeutet, in Gegenwart einer Base bzw. nach Deprotonierung des Thioglykolsäureesters mit einer Base.

Die Umsetzung von VI mit VII erfolgt auf eine dem Fachmann geläufige Weise, beispielsweise so, wie sie nachfolgend in den Beispielen beschrieben ist. Die Herstellung der Ketale der Ketone IV erfolgt ebenfalls auf eine dem Fachmann vertraute Weise, beispielsweise durch Umsetzung der Ketone IV mit Orthoameisensäureestern.

Die folgenden Beispiele erläutern die Erfindung näher. Fp. steht für Schmelzpunkt, Kp. für Siedepunkt.

### Beispiele

### 1. 4-(2,6-Dichloranilino)-3-thiophenessigsäure

3.46 g (0,01 mol) 3-(2,6-Dichloranilino)-2-hydroxycarbonyl-4-thiophenessigsäure werden in 30 ml 2N Salzsäure suspendiert und unter Rückfluß erhitzt, bis nach 2 h keine CO₂ Entwicklung mehr beobachtet wird. Das Reaktionsgemisch wird abgekühlt, zweimal mit Essigester extrahiert, die vereinigten organischen Phasen werden über Natriumsulfat getrocknet, filtriert und eingeengt. Umkristallisation aus Essigester liefert 2,84 g (94 %) der Titelverbindung (Fp.: 178°C).

Analog erhält man durch Decarboxylieren von
3-(2,6-Difluoranilino)-2-hydroxycarbonyl-4-thiophenessigsäure,
3-(3-Chlor-2-methylanilino)-2-hydroxycarbonyl-4-thiophenessigsäure,
3-(2-Chlor-6-methylanilino)-2-hydroxycarbonyl-4-thiophenessigsäure bzw.
3-(6-Brom-3-chlor-2-methylanilino)-2-hydroxycarbonyl-4-thiophenessigsäure
3-(2,6-Difluoranilino)-4-thiophenessigsäure (Fp.: 151°C),
3-(3-Chlor-2-methylanilino)-4-thiophenessigsäure (Fp.: 126 - 128°C),
3-(2-Chlor-6-methylanilino)-4-thiophenessigsäure (Fp.: 168 - 169°C) und
3-(6-Brom-3-chlor-2-methylanilino)-4-thiophenessigsäure

### 2. 3-(2,6-Dichloranilino)-2-hydroxycarbonyl-4-thiophenessigsäure

10 g (26,7 mmol) 3-(2,6-Dichloranilino)-2-methoxycarbonyl-4-thiophenessigsäuremethylester werden in 60 ml 1N Natronlauge bei 80°C 3 h lang gerührt. Die entstandene klare Lösung wird mit Salzsäure angesäuert, mit Essigester extrahiert, die organische Lösung wird getrocknet und zur Trockene eingeengt. Umkristallisation aus Essigester liefert 8,94 g (96,7%) der Titelverbindung (Fp.: 177°C).

Analog erhält man durch Verseifen von
3-(2,6-Difluoranilino)-2-methoxycarbonyl-4-thiophenessigsäuremethylester,
3-(3-Chlor-2-methylanilino)-2-methoxycarbonyl-4-thiophenessigsäuremethylester,
3-(2-Chlor-6-methylanilino)-2-methoxycarbonyl-4-thiophenessigsäuremethylester bzw.
3-(6-Brom-3-chlor-2-methylanilino)-2-methoxycarbonyl-4-thiophenessigsäuremethylester
3-(2,6-Difluoranilino)-2-hydroxycarbonyl-4-thiophenessigsäure (Fp.: 189 - 191°C),
3-(3-Chlor-2-methylanilino)-2-hydroxycarbonyl-4-thiophenessigsäure (Fp.: 157 - 159°C),
3-(2-Chlor-6-methylanilino)-2-hydroxycarbonyl-4-thiophenessigsäure (Fp.: 164°C) und
3-(6-Brom-3-chlor-2-methylanilino)-2-hydroxycarbonyl-4-thiophenessigsäure (Fp.: 178°C).

### 3. 3-(2,6-Dichloranilino)-2-methoxycarbonyl-4-thiophenessigsäuremethylester

Zu einer Lösung von 39 g (0,1 mol) 3-(2,6-Dichlorphenylimino)-tetrahydro-2-methoxycarbonyl-4-thiophenessigsäuremethylester in 250 ml Dichlormethan werden bei -20°C im Verlauf von 15 Min. 5,3 ml Brom in 10 ml Dichlormethan zugetropft. Das entstandene breiartige Reaktionsgemisch wird mit einer wässrigen Natriumsulfitlösung versetzt, die organische Phase abgetrennt, mit Wasser gewaschen, getrocknet und eingeengt. Umkristallisation des Rückstandes aus Methanol liefert 30 g der Titelverbindung (Fp.: 125 - 128°C).

Analog erhält man aus
3-(2,6-Difluorphenylimino)-tetrahydro-2-methoxycarbonyl-4-thiophenessigsäuremethylester,
3-(2-Chlor-6-methylphenylimino)-tetrahydro-2-methoxycarbonyl-4-thiophenessigsäuremethylester,
3-(3-Chlor-2-methylphenylimino)-tetrahydro-2-methoxycarbonyl-4-thiophenessigsäuremethylester bzw.
3-(6-Brom-3-chlor-2-methylphenylimino)-tetrahydro-2-methoxycarbonyl-4-thiophenessigsäuremethylester,
3-(2,6-Difluoranilino)-2-methoxycarbonyl-4-thiophenessigsäuremethylester (Fp.: 71 - 72°C),
3-(2-Chlor-6-methylanilino)-2-methoxycarbonyl-4-thiophenessigsäuremethylester (Fp.: 119 - 120°C),
3-(3-Chlor-2-methylanilino)-2-methoxycarbonyl-4-thiophenessigsäuremethylester (Öl) und
3-(6-Brom-3-chlor-2-methylanilino)-2-methoxycarbonyl-4-thiophenessigsäuremethylester (Öl).

### 4. 3-(2,6-Dichlorphenylimino)-tetrahydro-2-methoxycarbonyl-4-thiophenessigsäuremethylester

39 g (0,166 mol) 5-Carboxymethyl-tetrahydro-4-oxo-3-thiophenessigsäuremethylester und 27 g (0,166 mol) 2,6-Dichloranilin werden in Gegenwart von 1 g Toluolsulfonsäure in 150 ml Dichlorethan am Wasserabscheider gekocht, wobei wenig Wasser abgeschieden wird. Das Reaktionsgemisch wird anschließend mit 2N Natronlauge und dann mit Wasser gewaschen und eingeengt. Das Rohprodukt kann ohne weitere Reinigung weiterverarbeitet werden. Destillation mit einer Kurzwegdestille liefert 31 g (51 % d.Th. der Titelverbindung) (Kp.: 160 - 170°C / 1 Pa).

Durch Kondensation von 5-Carboxymethyl-tetrahydro-4-oxo-3-thiophenessigsäuremethylester mit 2,6 Difluoranilin, 2-Chlor-6-methylanilin, 3-Chlor-2-methylanilin bzw. 6-Brom-3-chlor-2-methylanilin erhält man
3-(2,6-Difluorphenylimino)-tetrahydro-2-methoxycarbonyl-4-thiophenessigsäuremethylester (Kp.: 150 - 160°C / 1 Pa), das beim Abkühlen kristallisiert (Fp.: 93 - 96°C),
3-(2-Chlor-6-methylphenylimino)-tetrahydro-2-methoxycarbonyl-4-thiophenessigsäuremethylester (Kp.: 140 - 150°C / 1 Pa),
3-(3-Chlor-2-methylphenylimino)-tetrahydro-2-methoxycarbonyl-4-thiophenessigsäuremethylester (Kp.: 142 - 152°C / 1 Pa) und
3-(6-Brom-3-chlor-2-methylphenylimino)-tetrahydro-2-methoxycarbonyl-4-thiophenessigsäuremethylester (Kp.: 155 - 163°C / 1 Pa).

### 5. 5-Carboxymethyl-tetrahydro-4-oxo-3-thiophenessigsäuremethylester

Zu 380 ml einer 5,25 M Natriummethylatlösung in Methanol werden 178,6 ml Thioglykolsäuremethylester bei 10°C zugetropft. Anschließend werden bei der gleichen Temperatur 310,8 g Itaconsäuremethylester zugetropft. Das auf ein Volumen von etwa 750 ml eingeengte Reaktionsgemisch wird unter gutem Rühren zu einer Lösung von 240 g CaCl₂ in 450 ml Wasser zugetropft. Das ausgefallene Calciumdi-(4,5-dihydro-2-methoxy-carbonyl-4-methoxycarbonylmethyl-3-thienyloxid) wird abgesaugt und mit wenig eiskaltem Wasser gewaschen. Der feuchte Rückstand wird mit überschüssiger 2 N Salzsäure behandelt, der entstehende Ester wird mit Ethylacetat extrahiert, die organische Phase wird getrocknet und im Vakuum eingeengt. Als Rückstand erhält man 352 g (77,7 %) der Titelverbindung als Öl.

## Patentansprüche

1. Verbindungen der Formel I 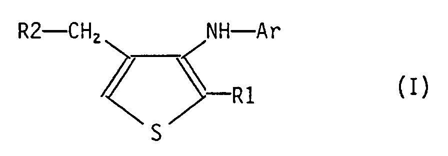 worin
R1 -CO-O-R6 bedeutet,
R2 -CO-O-R7 oder ein sonstiges funktionelles Carbonsäurederivat G bedeutet,
Ar den Rest 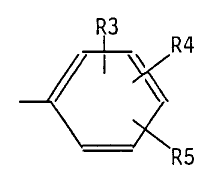 bedeutet,
R3 Wasserstoff, Halogen, 1-4C-Alkyl, 1-4C-Alkoxy oder Trifluormethyl bedeutet,
R4 Wasserstoff, Halogen, 1-4C-Alkyl oder 1-4C-Alkoxy bedeutet,
R5 Wasserstoff, Halogen, 1-4C-Alkyl oder 1-4C-Alkoxy bedeutet,
R6 Wasserstoff oder 1-4C-Alkyl bedeutet und
R7 Wasserstoff, 1-4C-Alkyl oder Hydroxy-1-4C-alkoxy-1-4C-alkyl bedeutet,
und die Salze dieser Verbindungen.

2. Verbindungen der Formel I nach Anspruch 1, worin
R1 -CO-O-R6 bedeutet,
R2 -CO-O-R7 bedeutet,
Ar 2,6-Dichlorphenyl, 2,6-Difluorphenyl, 2-Chlor-6-methylphenyl , 6-Brom-3-chlor-2-methylphenyl, 3-Chlor-2-methylphenyl, 2,6-Dichlor-3-methylphenyl, 2-Chlor-3-methylphenyl, 2,6-Dimethylphenyl, 2-Chlorphenyl, 4-Methoxy-2-methylphenyl oder 3-Trifluormethylphenyl bedeutet,
R6 Wasserstoff, Methyl oder Ethyl bedeutet und
R7 Wasserstoff, Methyl, Ethyl oder 2-Hydroxy-ethoxy-ethyl bedeutet,
und ihre Salze.

3. Verbindungen der Formel I nach Anspruch 1, worin
R1 -CO-O-R6 bedeutet,
R2 -CO-O-R7 bedeutet,
Ar 2,6-Dichlorphenyl bedeutet,
R6 Wasserstoff, Methyl oder Ethyl bedeutet und
R7 Wasserstoff, Methyl, Ethyl oder 2-Hydroxy-ethoxy-ethyl bedeutet,
und ihre Salze.

4. Verwendung von Verbindungen der Formel I nach Anspruch 1 zur Herstellung von Verbindungen der Formel II, 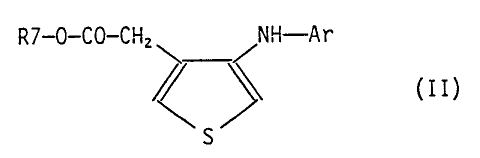 worin Ar und R7 die in Anspruch 1 genannten Bedeutungen haben, dadurch gekennzeichnet, daß man Verbindungen der Formel I nach Anspruch 1 decarboxyliert oder verseift und anschliessend decarboxyliert.

5. Verwendung von Verbindungen der Formel I nach Anspruch 2 zur Herstellung von Verbindungen der Formel II gemäß Anspruch 4, worin Ar und R7 die in Anspruch 2 genannten Bedeutungen haben, dadurch gekennzeichnet, daß man Verbindungen der Formel I nach Anspruch 2 decarboxyliert oder verseift und anschliessend decarboxyliert.

6. Verwendung von Verbindungen der Formel I nach Anspruch 3 zur Herstellung von Verbindungen der Formel II gemäß Anspruch 4, worin Ar und R7 die in Anspruch 3 genannten Bedeutungen haben, dadurch gekennzeichnet, daß man Verbindungen der Formel I nach Anspruch 3 decarboxyliert oder verseift und anschliessend decarboxyliert.

7. Verwendung der Verbindung der Formel I gemäß Anspruch 1, worin R1 -CO-O-R6, R2 -CO-O-R7, Ar 2,6 Dichlorphenyl, R6 Wasserstoff und R7 Wasserstoff bedeutet, zur Herstellung der Verbindung der Formel II gemäß Anspruch 4, worin Ar 2,6 Dichlorphenyl und R7 Wasserstoff bedeutet, dadurch gekennzeichnet, daß man die Verbindung I decarboxyliert.

8. Verfahren zur Herstellung der Verbindungen der Formel I nach Anspruch 1 und ihrer Salze, dadurch gekennzeichnet, daß man Verbindungen der Formel III oder ihre Salze, 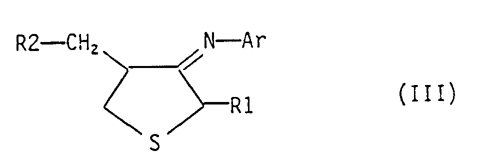 worin R1, R2 und Ar die in Anspruch 1 genannten Bedeutungen haben, dehydriert und gewünschtenfalls anschließend erhaltene Salze in die freien Verbindungen oder erhaltene freie Verbindungen in die Salze überführt.

9. Verfahren zur Herstellung der Verbindungen der Formel I nach Anspruch 2 und ihrer Salze, dadurch gekennzeichnet, daß man Verbindungen der Formel III gemäß Anspruch 8 oder ihre Salze, worin R1, R2 und Ar die in Anspruch 2 genannten Bedeutungen haben, dehydriert und gewünschtenfalls anschließend erhaltene Salze in die freien Verbindungen oder erhaltene freie Verbindungen in die Salze überführt.

10. Verfahren zur Herstellung der Verbindungen der Formel I nach Anspruch 3 und ihrer Salze, dadurch gekennzeichnet, daß man Verbindungen der Formel III gemäß Anpruch 8 oder ihre Salze, worin R1, R2 und Ar die in Anspruch 3 genannten Bedeutungen haben, dehydriert und gewünschtenfalls anschließend erhaltene Salze in die freien Verbindungen oder erhaltene freie Verbindungen in die Salze überführt.

## Claims

1. Compounds of the formula I 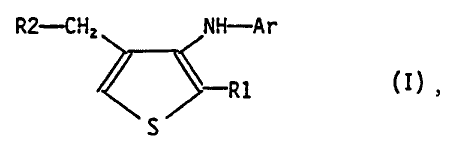 wherein
R1 is -CO-O-R6,
R2 is -CO-O-R7 or another functional carboxylic acid derivative G,
Ar is the radical 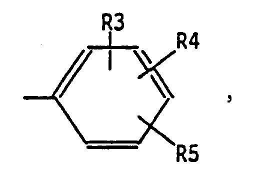
R3 is hydrogen, halogen, 1-4C-alkyl, 1-4C-alkoxy or trifluoromethyl,
R4 is hydrogen, halogen, 1-4C-alkyl or 1-4C-alkoxy,
R5 is hydrogen, halogen, 1-4C-alkyl or 1-4C-alkoxy,
R6 is hydrogen or 1-4C-alkyl and
R7 is hydrogen, 1-4C-alkyl or hydroxy-1-4C-alkoxy-1-4C-alkyl,
and the salts of these compounds.

2. Compounds of the formula I according to Claim 1, wherein
R1 is -CO-O-R6,
R2 is -CO-O-R7,
Ar is 2,6-dichlorophenyl, 2,6-difluorophenyl, 2-chloro-6-methylphenyl, 6-bromo-3-chloro-2-methylphenyl, 3-chloro-2-methylphenyl, 2,6-dichloro-3-methylphenyl, 2-chloro-3-methylphenyl, 2,6-dimethylphenyl, 2-chlorophenyl, 4-methoxy-2-methylphenyl or 3-trifluoromethylphenyl,
R6 is hydrogen, methyl or ethyl and
R7 is hydrogen, methyl, ethyl or 2-hydroxyethoxyethyl,
and their salts.

3. Compounds of the formula I according to Claim 1, wherein
R1 is -CO-O-R6,
R2 is -CO-O-R7,
Ar is 2,6-dichlorophenyl,
R6 is hydrogen, methyl or ethyl and
R7 is hydrogen, methyl, ethyl or 2-hydroxyethoxyethyl,
and their salts.

4. Use of compounds of the formula I according to Claim 1 for the preparation of compounds of the formula II 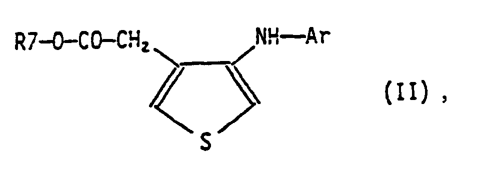 wherein Ar and R7 have the meanings mentioned in Claim 1, characterized in that compounds of the formula I according to Claim 1 are decarboxylated or hydrolysed and then decarboxylated.

5. Use of compounds of the formula I according to Claim 2 for the preparation of compounds of the formula II according to Claim 4, wherein Ar and R7 have the meanings mentioned in Claim 2, characterized in that compounds of the formula I according to Claim 2 are decarboxylated or hydrolysed and then decarboxylated.

6. Use of compounds of the formula I according to Claim 3 for the preparation of compounds of the formula II according to Claim 4, wherein Ar and R7 have the meanings mentioned in Claim 3, characterized in that compounds of the formula I according to Claim 3 are decarboxylated or hydrolysed and then decarboxylated.

7. Use of the compound of the formula I according to Claim 1, wherein R1 is -CO-O-R6, R2 is -CO-O-R7, Ar is 2,6-dichlorophenyl, R6 is hydrogen and R7 is hydrogen, for the preparation of the compound of the formula II according to Claim 4, wherein Ar is 2,6-dichlorophenyl and R7 is hydrogen, characterized in that the compound I is decarboxylated.

8. Process for the preparation of the compounds of the formula I according to Claim 1 and their salts, characterized in that compounds of the formula III or their salts 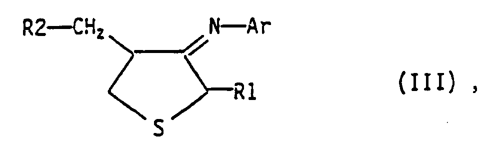 wherein R1, R2 and Ar have the meanings mentioned in Claim 1, are dehydrogenated and, if desired, salts obtained are then converted into the free compounds or free compounds obtained are converted into the salts.

9. Process for the preparation of the compounds of the formula I according to Claim 2 and their salts, characterized in that compounds of the formula III according to Claim 8 or their salts, wherein R1, R2 and Ar have the meanings mentioned in Claim 2, are dehydrogenated and, if desired, salts obtained are then converted into the free compounds or free compounds obtained are converted into the salts.

10. Process for the preparation of the compounds of the formula I according to Claim 3 and their salts, characterized in that compounds of the formula III according to Claim 8 or their salts, wherein R1, R2 and Ar have the meanings mentioned in Claim 3, are dehydrogenated and, if desired, salts obtained are then converted into the free compounds or free compounds obtained are converted into the salts.

## Revendications

1. Composés de formule I 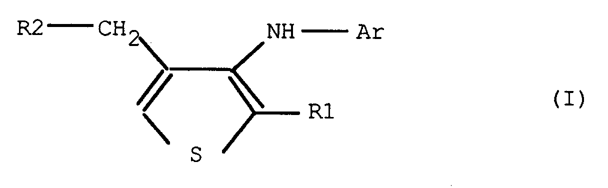 dans laquelle
R1 est un radical -CO-O-R6,
R2 est un radical -CO-O-R7 ou un autre dérivé fonctionnel G d'un acide carboxylique,
Ar est le résidu 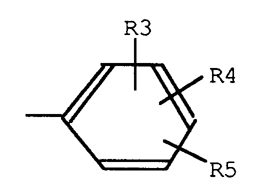 R3 est un hydrogène ou un radical halogéno, alkyle en C₁-C₄, alcoxy en C₁-C₄ ou trifluorométhyle,
R4 est un hydrogène ou un radical halogéno, alkyle en C₁-C₄ ou alcoxy en C₁-C₄,
R5 est un hydrogène ou un radical halogéno, alkyle en C₁-C₄ ou alcoxy en C₁-C₄,
R6 est un hydrogène ou un radical alkyle en C₁-C₄, et
R7 est un hydrogène ou un radical alkyle en C₁-C₄ ou hydroxy-(alcoxy en C₁-C₄)-(alkyle en C₁-C₄), et les sels de ces composés.

2. Composés de formule I selon la revendication 1, dans laquelle
R1 est un radical -CO-O-R6,
R2 est un radical -CO-O-R7,
Ar est le radical 2,6-dichlorophényle, 2,6-difluorophényle, 2-chloro-6-méthylphényle, 6-bromo-3-chloro-2-méthylphényle, 3-chloro-2-méthylphényle, 2,6-dichloro-3-méthylphényle, 2-chloro-3-méthylphényle, 2,6-diméthylphényle, 2-chlorophényle, 4-méthoxy-2-méthylphényle ou 3-trifluorométhylphényle,
R6 est un hydrogène ou le radical méthyle ou éthyle, et
R7 est un hydrogène ou le radical méthyle, éthyle ou 2-hydroxyéthoxyéthyle,
et leurs sels.

3. Composés de formule I selon la revendication 1, dans laquelle
R1 est un radical -CO-O-R6,
R2 est un radical -CO-O-R7,
Ar est le radical 2,6-dichlorophényle,
R6 est un hydrogène ou le radical méthyle ou éthyle, et
R7 est un hydrogène ou le radical méthyle, éthyle ou 2-hydroxyéthoxyéthyle, et leurs sels.

4. Utilisation de composés de formule I selon la revendication 1 pour préparer des composés de formule II 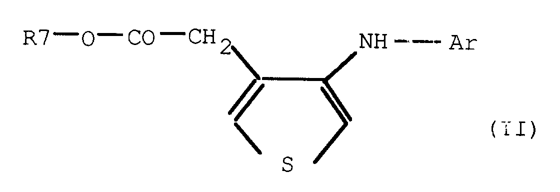 dans laquelle Ar et R7 ont les signification données dans la revendication 1, caractérisée en ce qu'on soumet à une décarboxylation, ou à une saponification suivie d'une décarboxylation, les composés de formule I selon la revendication 1.

5. Utilisation de composés de formule I selon la revendication 2 pour préparer des composés de formule II selon la revendication 4, où Ar et R7 ont les significations données dans la revendication 2, caractérisée en ce qu'on soumet les composes de formule I selon la revendication 2 à une décarboxylation, ou à une saponification suivie d'une décarboxylation.

6. Utilisation de composés de formule I selon la revendication 3 pour préparer des composés de formule II selon la revendication 4, où Ar et R7 ont les significations données dans la revendication 3, caractérisée en ce qu'on soumet les composés de formule I selon la revendication 3 à une décarboxylation ou à une saponification suivie d'une décarboxylation.

7. Utilisation du composé de formule I selon la revendication 1, dans laquelle R1 est un radical -CO-O-R6, R2 est un radical -CO-O-R7, Ar est le radical 2,6-dichlorophényle, R6 est un hydrogène et R7 est un hydrogène, pour préparer le composé de formule II selon la revendication 4 dans laquelle Ar est le radical 2,6-dichlorophényle et R7 est un hydrogène, caractérisée en ce qu'on soumet le composé I à une décarboxylation.

8. Procédé pour préparer les composés de formule I selon la revendication 1 et leurs sels, caractérisé en ce qu'on procède à une déshydrogénation des composés de formule III ou de leurs sels 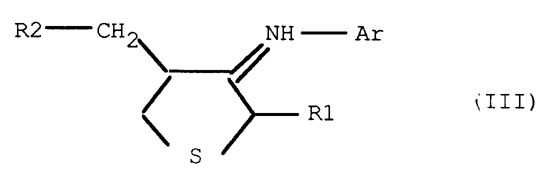 dans laquelle R1, R2 et Ar ont les signification données dans la revendication 1, puis, si on le souhaite, qu'on convertit les sels obtenus en les composes libres, ou les composés libres obtenus en les sels.

9. Procédé pour préparer les composés de formule I selon la revendication 2 et leurs sels, caractérisé en ce qu'on soumet à une déshydrogénation les composés de formule III selon la revendication 8 ou leurs ses, dans laquelle R1, R2 et Ar ont les significations données dans la revendication 2, et, si on le souhaite, on convertit les sels obtenus en les composés libres ou les composés libres obtenus en les sels.

10. Procédé pour préparer les composes de formule I selon la revendication 3 et leurs sels, caractérisé en ce qu'on soumet à une déshydrogénation les composes de formule III selon la revendication 8 ou leurs sels dans laquelle R1, R2 et Ar ont les significations données dans la revendication 3, et que, si on le souhaite on convertit ensuite les sels obtenus en les composés libres, ou les composés libres obtenus en les sels.
